# EUROPÄISCHE PATENTANMELDUNG

(11) **EP 2 133 080 A1**
(43) Veröffentlichungstag der Anmeldung: **16.12.2009**
(21) Anmeldenummer: 08158209.0
(22) Anmeldetag: 13.06.2008
(51) Int. Cl.: A61K 31/56, A61K 31/35, A61K 36/48, A61K 36/55, A61K 36/71, A61K 36/185, A61K 36/85

(54) **Zusammensetzungen enthaltend Equol**

(71) Anmelder: Haelan Schweiz GmbH, 8808 Pfäffikon SZ (CH)
(72) Erfinder: Schmid, Juerg Daniel, 8004 Zürich (CH)
(74) Vertreter: Steglich, Gregor

(57) **Zusammenfassung**

Gegenstand der Erfindung sind Zusammensetzungen enthaltend gereinigtes Equol und mindestens ein weiteres Phytoestrogen, sowie Verwendungen dieser Zusammensetzungen, Verfahren zu deren Herstellung und Nahrungsergänzungsmittel, Nahrungsmittel und Arzneimittel, die solche Zusammensetzungen enthalten.

## Beschreibung

Gegenstand der Erfindung sind Zusammensetzungen enthaltend gereinigtes Equol und mindestens ein weiteres Phytoestrogen, sowie Verwendungen dieser Zusammensetzungen, Verfahren zu deren Herstellung und Nahrungsergänzungsmittel, Nahrungsmittel und Arzneimittel, die solche Zusammensetzungen enthalten.

Isoflavone, auch Isoflavonoide genannt, sind meist gelblich gefärbte Pflanzenfarbstoffe, die als Derivate des Isoflavons zur Klasse der Flavonoide zählen. Sie dienen unter anderem der pflanzlichen Abwehr von Pathogenen. Einige bekanntere Isoflavone sind Daidzein, das als Glucosid Daidzein in Sojamehl vorkommt, und Genistein aus Sojabohnen und Rotklee.

Isoflavonoide weisen wie auch Lignane wegen ihrer chemisch-strukturellen Ähnlichkeit zu den 17-Ketosteroiden (Estrogene, Androgene) in hohen Dosen eine schwache geschlechtshormonelle Wirkung auf den Menschen. Sie sind daher Phytoestrogene.

Isoflavone werden unter anderem als Nahrungsergänzungsmittel und in der Alternativmedizin für viele verschiedene Anwendungen eingesetzt. So wird der Verzehr von Flavonoiden bzw. Soja-Präparaten in den Wechseljahren empfohlen, allgemein gegen Altersbeschwerden, und auch zur Vorbeugung von hormonanhängigen Krebserkrankungen wie Brust- und Prostatakrebs.

Zahlreiche Studien belegen die gesundheitsfördernden Wirkungen von Isoflavonen. So wurde wiederholt bei Einnahme von Sojaextrakten, Sojaprotein-Isolaten bzw. Isoflavonen eine deutliche Verbesserung von menopausalen Beschwerden und eine Senkung menstruationsbedingter Migräne gezeigt. Aus weiteren Studien resultiert, dass Isoflavone die Aktivität der Osteoblasten fördern, die der Osteoklasten hemmen und insgesamt das Risiko für den Knochenverlust reduzieren. Eine Verwendung von Isoflavonen aus Soja bei Beschwerden im Zusammenhang mit der Menopause wird beispielsweise in EP1481676 B1 beschrieben.

Das Isoflavan Equol (4',7-Dihydroxyisoflavan) wird aus dem Isoflavon Daidzein nach der Einnahme in der Darmflora gebildet. Equol gehört somit der Gruppe der sekundären Pflanzenstoffe an. Es wird vermutet, dass die Umwandlung durch Streptokokken, Milchsäurebakterien und Bifidobakterien erfolgt. Equol ist nach dem Verzehr von Daidzein-reichen Lebensmitteln wie Soja in Blut und Harn nachweisbar. Equol besitzt eine leichte Estrogenaktivität (0,1% der Aktivität von Steroid-Estrogenen) und kann an die Estrogenrezeptoren ERα und ERβ binden. Nur etwa ein Drittel (kaukasische Bevölkerung) bis die Hälfte (Japaner) der Menschen können aus Daidzein Equol bilden. Bei den "Equolbildnern" ist der Cholesterin senkende und entzündungshemmende Effekt einer sojareichen Ernährung höher als bei Personen, die kein Equol bilden können.

Das EP1025850 betrifft eine Zusammensetzung, in der Equol mikrobiologisch aus Isoflavonen aus Soja hergestellt wird, sowie deren Verwendung zur Behandlung von Beschwerden im Zusammenhang mit der Menopause. Mikrobiologische Herstellungsprozesse weisen jedoch allgemein Nachteile auf. So sind sie relativ störanfällig, weil sich Mikroorganismen verändern und dadurch veränderte Produkte erzeugen können. Die Herstellung muss daher kontinuierlich überwacht werden. Gegebenenfalls müssen die Mikroorganismen nach der Herstellung getötet und abgetrennt werden.

Die WO2004/009035 beschreibt Verfahren zur Herstellung von Equol durch organische Synthese oder mikrobiologische Prozesse. Es werden auch Zusammensetzungen offenbart, in denen Equol mikrobiologisch aus Daidzein hergestellt wird.

Das EP079555381 betrifft die Herstellung von mit Isoflavonen angereicherten Fraktionen aus Sojabohnen.

Verfahren zur Herstellung von Enantiomeren-reinem Equol werden auch in der und WO00/049009, WO2007/016423, US2007/014788A1 und Wang et al., 2005, Appl. and Env. Microbiol., 71, 214-219 offenbart.

Eine Übersicht zum Stand der Technik zu Wirkungen von Soja und Isoflavonen aus Soja wird von Kotecha und Lockwood in The Pharmaceut. J. 2005, 275, S. 483-487 gegeben.

### Problem:

Der Erfindung liegt das Problem zugrunde, Mittel bereitzustellen, die die oben beschriebenen Nachteile vermeiden. Ein Nachteil von bekannten Phytoestrogenen ist, dass diese häufig nur eine geringe Wirksamkeit aufweisen. Es sollen daher Nahrungsergänzungsmittel und Arzneimittel auf der Basis von Phytotherapeutika oder Phytoestrogenen bereitgestellt werden, die eine verbesserte Wirksamkeit aufweisen. Die Mittel sollen dabei möglichst auf einfache Weise verfügbar sein und den Konsumenten möglichst wenig belasten.

Der Erfindung liegt insbesondere das Problem zugrunde, Mittel zur Linderung oder Beseitigung von Beschwerden im Zusammenhang mit dem Klimakterium (Menopause) bereitzustellen. Das Mittel soll dabei in den verschiedenen Phasen der Menopause und gegen ein breites Spektrum von Beschwerden wirken. Die Mittel sollen auch bei Beschwerden einsetzbar sein, die nicht im Zusammenhang mit dem Klimakterium stehen.

### Lösung:

Das erfindungsgemäße Problem wird überraschenderweise gelöst durch Zusammensetzungen und deren Verwendung gemäß den Ansprüchen 1 bis 15.

Gegenstand der Erfindung ist eine Zusammensetzung, enthaltend
(a) gereinigtes Equol und
(b) mindestens ein weiteres Phytoestrogen.

Das Equol wird der Zusammensetzung bei deren Herstellung in gereinigter Form zugesetzt. Verfahren zur Reinigung und zur Herstellung von reinem Equol, dem Racemat oder der Enantiomeren sind bekannt. So kann Equol durch organische Synthese, mikrobiologisch oder enzymatisch hergestellt werden. Entsprechende Verfahren werden in WO2004/009035A2, WO2007/016423, EP0267155, US2007/0149788, EP0795553 und Wang et al., 2005, Appl. and Env. Microbiol., 71, 214-219 beschrieben, auf die hier ausdrücklich Bezug genommen wird. Das gereinigte Equol weist bevorzugt eine Reinheit von mindestens 90, 95, 98 oder 99 Gew.-% auf.

In einer bevorzugten Ausführungsform der Erfindung enthält die Zusammensetzung 0,5 bis 30 Gew.-%, bevorzugt 1 bis 15 oder 2 bis 10 Gew.-% gereinigtes Equol. In weiteren bevorzugten Ausführungsformen sind mindestens 0,5, 1, 2 oder 3 Gew.-% Equol enthalten.

Das Equol ist ein essentieller Bestandteil der Zusammensetzung, da es in synergistischer Weise die Wirkung der weiteren Komponenten verbessert. Diese Wirkung konnte anhand des Estrogenspiegels gezeigt werden. Erfindungsgemäß kann eine höhere Menge Equol eingesetzt werden als in Zusammensetzungen, in denen Equol durch Mikroorganismen aus Extrakten von Isoflavonen hergestellt wird, da in solchen Gemischen nur wenige Isoflavone wie Daidzein umsetzbar sind. Das erfindungsgemäße Bereitstellen einer Zusammensetzung mit gereinigtem Equol ist auch einfacher, genauer und weniger störanfällig als die bekannte Herstellung von Equol in der Zusammensetzung selbst durch Mikroorganismen.

In einer Ausführungsform der Erfindung enthält die Zusammensetzung ausschließlich R-Equol oder S-Equol. Dabei bedeutet "ausschließlich", dass das jeweilige Enantiomer beispielsweise mindestens 95, 98 oder 99,5 Gew.-% des enthaltenen Equols ausmacht. Da bekannt ist, dass für die Linderung mancher Beschwerden nur oder verstärkt die S- oder die R-Form verantwortlich ist, kann so die Wirkung der Zusammensetzung gezielt verbessert werden. Es ist bekannt, das in der menschlichen Darmflora vorwiegend das S-Equol synthetisiert wird, das auch für die meisten bekannten vorteilhaften Wirkungen verantwortlich ist (Setchell et al., 2005, Am. J. Clin. Nutr. 81, 1072-9). Es ist daher bevorzugt, in der erfindungsgemäßen Zusammensetzung S-Equol einzusetzen. In einer weiteren Ausführungsform wird das Racemat eingesetzt.

Die erfindungsgemäße Zusammensetzung enthält mindestens ein weiteres Phytoestrogen (b). Phytoestrogene ähneln strukturell den endogenen Estrogenen, insbesondere dem 17β-Estradiol, und weisen ähnliche Wirkungen auf, jedoch im Allgemeinen mit deutlich geringerer Aktivität. Phytoestrogene sind insbesondere in der Lage, mit den α- und β-Estrogen-Rezeptoren zu wechselwirken. Sie weisen häufig phenolische Strukturelemente auf und sind oft Polyphenole. Es sind auch Phytoestrogene ohne phenolische Gruppen bekannt, beispielsweise Triterpene. Bevorzugt sind die Phytoestrogene (b) Polyphenole und/oder Triterpene.

Polyphenole haben allgemein ein großes und breites Wirkspektrum für klimakterische Beschwerden. Sie können antioxidativ, antiviral, antimikrobiell und antikarzinogen wirken, Entzündungen und Allergien hemmen und das Immunsystem modulieren. Verschiedene pharmakologische Studien belegen eine estrogenaktive Wirksamkeit von Polyphenolen (Newton et al., Maturitas 2005; 52(2), 134-146).

Phytoestrogene wie die Isoflavone zählen zu den selektiven Estrogen-Rezeptor-Modulatoren (phytoSERMs). Diese binden an den Estrogenrezeptor, allerdings mit einer etwa hundert- bis tausendmal schwächeren Aktivität als die endogenen Steroidestrogene. Damit ist grundlegend eine agonistische oder antagonistische Wirkung möglich. Zudem gibt es zwei Subtypen (ERα und ERβ) des Estrogenrezeptors, so dass die Effekte insgesamt komplex sind. Während 17-β-Estradiol an beide Rezeptoren gleich gut bindet, treten die Phytoestrogene mit deutlich höherer Affinität mit dem β-Rezeptor in Wechselwirkung. Diese Rezeptoren kommen im Körper in unterschiedlichen Geweben vor. ERα - der »klassische« Subtyp - ist bevorzugt in den Fortpflanzungsorganen, der Brust und der Niere zu finden. ERβ ist dagegen vorzugsweise im Gehirn, der Lunge, der Prostata, dem Knochengewebe und im Herz-Kreislaufsystem lokalisiert. Dadurch ergeben sich durch die PhytoSERMs positive Effekte besonders auf Knochen, Gehirn, das Herz-Kreislaufsystem und den Urogenitaltrakt. Die antiestrogene Wirkung der Phytoestrogene schließt antiproliferative Effekte auf Tumorzellen, die unter anderem durch die Hemmung des Mitoseenzyms Topoisomerase und des Enzyms Tyrosinkinase sowie die Hemmung der Angiogenese erklärbar sind, mit ein. Von Bedeutung ist in diesem Zusammenhang auch die Stimulation des SHBG (Sex-Hormone-Binding Globulin). Dieses Protein bindet Steroidhormone im Plasma und mindert dadurch die Konzentration an freien Estrogenen (und Androgenen). Neben den genregulierten Effekten der Estrogene werden auch nicht-genabhängige Wirkungen wie beispielsweise Regulation des Zellzyklus, antioxidative und antiinflammatorische Effekte für die Wirkung verantwortlich gemacht.

Geeignete Phytoestrogene (b) sind insbesondere Flavonoide, Isoflavonoide, Isoflavone, Lignane, Cumestane und Triterpene.

In einer bevorzugten Ausführungsform der Erfindung liegt das Phytoestrogen (b) in Form eines pflanzlichen Extraktes vor. Erfindungsgemäß steht der Begriff "Phytoestrogen" auch für Extrakte, die Phytoestrogene enthalten und somit eine estrogene Aktivität aufweisen. Erfindungsgemäß bedeutet "Extrakt", dass das Phytoestrogen oder ein Gemisch aus Phytoestrogenen aus einer pflanzlichen Quelle aufgereinigt und dadurch angereichert wird. Es ist bevorzugt, dass in dem Extrakt Phytoestrogene mindestens um das 2-, 5- oder 10fache gegenüber dem Ausgangsmaterial angereichert werden.

Die Extrakte können nach bekannten Verfahren erhalten werden, beispielsweise Mazeration, Dimazeration, Digestion, Re-/Perkolation, Soxhletverfahren, Turbo- (Wirbel), Ultra-, Turrax-, Ultraschall- und Gegenstrom-Extraktion, Fraktionierung. Pflanzliche Extrakte enthalten üblicherweise mehr oder weniger hohe Anteile weiterer Komponenten. Erfindungsgemäß einsetzbare Extrakte können auch weiterverarbeitet sein, beispielsweise durch chemische Modifikation oder Sterilisierung. Phytoestrogene Extrakte sind meist Vielstoffgemische, die in ihrer Gesamtheit wirken. In Präparaten wird oft eine Leitsubstanz angegeben, die allerdings nicht der Hauptwirkstoff sein muss. Leitsubstanzen dienen auch der Standardisierung von Extrakten.

In einer bevorzugten Ausführungsform ist das Phytoestrogen (b) ausgewählt aus Isoflavonen, insbesondere Extrakten davon aus Soja; Lignanen, insbesondere Extrakten davon aus Leinsamen; sowie Extrakten aus Traubensilberkerze, Granatapfel oder Mönchspfeffer, oder einem Gemisch zweier oder mehrerer dieser Substanzen.

In einer weiteren bevorzugten Ausführungsform ist als Phytoestrogen (b) ein Extrakt von Isoflavonen, insbesondere aus Soja, sowie mindestens ein weiteres Phytoestrogen (b), ausgewählt aus Extrakten aus Traubensilberkerze, Granatapfel oder Mönchspfeffer und Extrakten von Lignanen, insbesondere aus Leinsamen, enthalten.

In einer weiteren Ausführungsform der Erfindung sind als Phytoestrogene (b) enthalten:
(b1) ein pflanzlicher Extrakt von Isoflavonen,
(b2) ein pflanzlicher Extrakt von Lignanen und
(b3) mindestens ein weiterer Pflanzenextrakt, insbesondere ausgewählt aus Traubensilberkerze, Granatapfel und Mönchspfeffer.

Weitere geeignete Phytoestrogene (b) sind beispielsweise Extrakte aus Johanniskraut, Passionsfrucht, Ginseng, Lavendel, Melisse, Hopfen, Baldrian, Weizenkeimen und Lecithin.

Die erfindungsgemäße Zusammensetzung unterscheidet sich damit von bekannten Zusammensetzungen, bei denen aus einem Gemisch von Isoflavonen in Gegenwart von Mikroorganismen Equol aus Daidzein hergestellt wird. Solche Zusammensetzungen werden beispielsweise in der WO2004/009035 und EP1025850 beschrieben. Die erfindungsgemäße Zusammensetzung unterscheidet sich, weil erfindungsgemäß das Daidzein nicht durch Umsetzung zu Equol aufgebraucht wird. Darüber hinaus ist die Menge an Equol in der erfindungsgemäßen Zusammensetzung nicht durch die Menge an enthaltenem Daidzein limitiert. Im Gegensatz zu den bekannten Zusammensetzungen weist die erfindungsgemäße Zusammensetzung die Menge an Daidzein eines natürlichen Isoflavon-Extrakts auf und zusätzlich eine definierte Menge gereinigten Equols. Die erfindungsgemäße Zusammensetzung enthält vorzugsweise keine Enzyme oder Mikroorganismen, die Equol herstellen. Sie enthält bevorzugt keine lebenden oder toten Mikroorganismen. Die Zusammensetzung enthält vorzugsweise keine Enzyme, in isolierter Form oder als Bestandteil von Mikroorganismen, und keine Mikroorganismen, die Equol aus anderen Isoflavonen wie Daidzein herstellen. Die Zusammensetzung enthält vorzugweise auch keine solchen Enzyme oder Mirkoorganismen in inaktivierter Form, wie denaturierte Enzyme. In der erfindungsgemäßen Zusammensetzung findet daher im Wesentlichen keine Umsetzung von Isoflavonen zu Equol statt.

In einer bevorzugten Ausführungsform der Erfindung ist in der Zusammensetzung das Gewichtsverhältnis des gereinigten Equols zu dem Extrakt von Isoflavonen zwischen 1:10 und 5:1, bevorzugt zwischen 1:5 und 2:1, besonders bevorzugt zwischen 1:4 und 1:1.

In einer bevorzugten Ausführungsform der Erfindung ist zusätzlich mindestens ein Aminosäurederivat oder eine Aminosäure enthalten. Erfindungsgemäß geeignete Aminosäuren und Aminosäurederivate sind insbesondere solche, die eine physiologische Wirkung aufweisen. Mit Derivate werden Aminosäuren bezeichne, bei denen die Aminogruppe oder die Carboxylgruppe chemisch derivatisiert ist. So kann beispielsweise die Aminogruppe sekundär, tertiär oder quartär sein oder acyliert oder acetyliert sein oder die Carboxylgruppe verestert oder amidiert sein. Besonders bevorzugt sind L-Carnitin und dessen Derivate, wie Acetyl-N-Carnitin.

In einer bevorzugten Ausführungsform der Erfindung enthält die Zusammensetzung:
(a) 0,5 bis 30 Gew.-% gereinigtes Equol,
(b1) 1 bis 60 Gew.-% pflanzlicher Extrakt von Isoflavonen,
(b2) 1 bis 30 Gew.-% pflanzlicher Extrakt von Lignanen,
(b3) 1 bis 90 Gew.-% weitere Pflanzenextrakte und
(c) 0 bis 20 Gew.-% Aminosäurederivate.

In einer weiteren bevorzugten Ausführungsform der Erfindung enthält die Zusammensetzung
(a) 01 bis 15 Gew.-% gereinigtes Equol,
(b1) 2 bis 30 Gew.-% pflanzlicher Extrakt von Isoflavonen aus Soja,
(b2) 5 bis 40 Gew.-% pflanzlicher Extrakt von Lignanen aus Leinsamen,
(b3) 1 bis 30 Gew.-% Extrakt aus Traubensilberkerze,
(b4) 1 bis 30 Gew.-% Extrakt aus Mönchspfeffer,
(b5) 1 bis 30 Gew.-% Extrakt aus Granatapfel,
(c) 1 bis 15 Gew.-% Aminosäurederivate, insbesondere Acetyl-L-Carnitin.

In einer bevorzugten Ausführungsform der Erfindung ist die Zusammensetzung ein Nahrungsergänzungsmittel oder ein Arzneimittel.

Ein Nahrungsergänzungsmittel ist insbesondere ein Lebensmittel, das dazu bestimmt ist, die allgemeine Ernährung zu ergänzen. Es ist üblicherweise ein Konzentrat von Nährstoffen oder sonstigen Stoffen mit ernährungsspezifischer oder physiologischer Wirkung allein oder in Zusammensetzung. Als Nahrungsergänzungsmittel wird insbesondere die Produktgruppe bezeichnet, die im EU-Recht durch die Richtlinie 2002/46/EG geregelt ist und in der insbesondere zulässige Mineralstoffe und Vitamine aufgeführt sind. Nahrungsergänzungsmittel liegen bevorzugt in dosierter Form vor. Geeignete Formen sind insbesondere Kapseln, Pastillen, Tabletten, Pillen, Brausetabletten und anderen ähnlichen Darreichungsformen, Pulverbeutel, Flüssigampullen, Flaschen mit Tropfeinsätzen, Tuben und ähnlichen Darreichungsformen von Flüssigkeiten, Pasten und Pulvern. In einer bevorzugten Ausführungsform enthält eine dosierte Zusammensetzung 10 bis 200 g Equol, insbesondere 20 bis 100 oder 30 bis 70 mg Equol. Eine solche Dosis ist zur täglichen Verabreichung geeignet. In einer solchen dosierten Zusammensetzung können beispielsweise 10 bis 300 mg, insbesondere 30 bis 200 oder 40 bis 150 mg Isoflavone enthalten sein. Weiter können je 30 bis 1000, insbesondere je 100 bis 700 mg weiterer Pflanzenextrakte und/oder 20 bis 500, insbesondere 50 bis 300 mg Aminosäurederivate enthalten sein. Bevorzugt ist die Dosis des gesamten Nahrungsergänzungsmittels zwischen 100 mg und 5g, insbesondere zwischen 250 mg und 2,5 g.

Gegenstand der Erfindung ist auch ein Nahrungsmittel oder Arzneimittel, das eine erfindungsgemäße Zusammensetzung oder ein erfindungsgemäßes Nahrungsergänzungsmittel enthält. Das Nahrungsmittel kann eine flüssige oder feste Form aufweisen. Die Zusammensetzung kann beispielsweise Milchprodukten wie Joghurt, Molke, Milch und Quark, Brotaufstrichen, Fertiggerichten, Desserts oder Getränken wie Säften, Tee, Mineralwasser zugesetzt werden. Das Arzneimittel kann beispielsweise eine Tablette, Lösung oder Suspension sein.

### Isoflavone:

Die erfindungsgemäße Zusammensetzung enthält außerdem einen pflanzlichen Extrakt von Isoflavonen (b). Solche Extrakte sind beispielsweise aus Sojamehl, Sojabohnen, Kichererbsen, Rotklee und anderen Kleearten, Orobol, Sandelholz, Rotholz und anderen Hölzern isolierbar. Bevorzugt werden Extrakte eingesetzt, die Daidzein und/oder Daidzin enthalten.

Im erfindungsgemäßen Produkt werden bevorzugt Isoflavone aus Soja eingesetzt. Das darin enthaltene Daidzein wird teils im Darmtrakt zu Equol metabolisiert. Nicht resorbierte Isoflavone gelangen in den Dickdarm und können durch die Darmflora reduktiv metabolisiert werden. Dabei wird Daidzein zuerst zu Dihydrodaidzein reduziert, welches entweder durch Spaltung des C-Ringes zu O-Demethylangolensin oder unter Erhalt des C-Ringes zu dem Isoflavon Equol verstoffwechselt werden kann. Ein erfindungsgemäßer Extrakt enthält bevorzugt mindestens 50%, insbesondere mindestens 80, 90 oder 95 Gew.-% Isoflavone

Die Bildung von Equol ist starken individuellen Unterschieden unterworfen und vor allem von der Zusammensetzung der Darmflora abhängig. Nur etwa 30 % der westlichen Bevölkerung sind in der Lage, Equol aus Daidzein zu bilden. Dabei belegen Studien die Vorteile der Equolproduzenten. So sind Personen im Klimakterium beschwerden frei, wenn sie zu der Gruppe der Equolproduzenten gehören, während die Gruppe der Nichtequolproduzenten immer zu Beschwerden neigt (Menopause 2007, 14(5):866-874). In Asien ist die Gruppe der Equolproduzenten deutlich höher. Dies erklärt auch, warum der Hauptteil asiatischer Frauen keine Beschwerden im Klimakterium aufweist. Man geht davon aus, dass die Essgewohnheiten (Soja) der Asiaten dafür verantwortlich sind, dass die Anzahl der Equolproduzenten so hoch ist. Das erfindungsgemäße Produkt hat den Vorteil, dass Personen, die kein körpereigenes Equol metabolisieren, mit Equol versorgt werden.

Erfindungsgemäß können die Isoflavone Glykoside, Aglyka oder Gemische davon sein. Allgemein kommen Flavonoide häufig als lösliche Glykoside im Zellsaft von Pflanzen vor. Zuckerfreie Strukturen werden Aglyka genannt.

In einer bevorzugten Ausführungsform werden die Isoflavone aus Soja, insbesondere fermentiertem Soja, angereichert. In Sojabohnen sind hohe Anteile an Daidzein und Genistein und den β-Glycosiden Genistin und Daidzin enthalten. Es sind außerdem geringere Anteile an Glycitein und Glycitin enthalten.

Klinische Studien zwischen 1995 und 2008 ließen bei Verabreichung von Sojaextrakten, Sojaprotein-Isolaten bzw. Isoflavone, überwiegend eine deutliche Verbesserung der menopausalen Beschwerden erkennen. Weitere Studien lassen bei einer Supplementierung mit Isoflavonen auf eine Senkung menstruationsbedingter Migräne und einen Zusammenhang mit der Osteoporoseprävention schließen. Aus tierexperimentellen Studien resultiert, dass Isoflavone die Aktivität der Osteoblasten fördern, diejenige der Osteoklasten hemmen und insgesamt das Risiko für den Knochenverlust reduzieren. Die zugrunde liegenden Wirkmechanismen, betreffen die Anregung der Proliferation der Osteoblasten, deren Schutz vor oxidativer Schädigung und eine Erhöhung der Apoptose der Osteoklasten, wobei bevorzugt Knochen der Hüfte, spinale und lumbale Knochen sowie Knochen des Schenkelhalses profitierten.

Isoflavone zeigen auch einen positiven Einfluss auf Gefäße, Knochen und das zentrale Nervensystem (ZNS). Zudem wird angenommen, dass sie die Hemmung der Lipidperoxidation (antioxidative Wirkung), die günstige Beeinflussung des Lipidprofils, die Hemmung der Proliferation endothelialer Muskelzellen, die Freisetzung von NO, die Erhaltung der Gefäßelastizität, eine positive Beeinflussung von Insulinresistenz, Hyperglykämie und Lipidstoffwechsel bewirken. Neben hämodynamischer und antioxidativer Effekte werden auch Wirkungen auf den neuronalen Transmitterhaushalt diskutiert, welche für die in Humanstudien beobachtete Verbesserung der mentalen Leistungsbereitschaft (Kurz-, Langzeitgedächtnis, Reaktivität) verantwortlich gemacht werden.

### Lignane

In einer bevorzugten Ausführungsform der Erfindung enthält die erfindungsgemäße Zusammensetzung einen pflanzlichen Extrakt von Lignanen. Lignane sind Phytoestrogene und Phytonährstoffe, die in unraffiniertem Getreide, Hülsenfrüchten, bestimmten Gemüsesorten und Samen, wie Leinsamen, vorkommen. Sie sind Dimere von Phenylpropanderivaten, die über das C-2 der Propanseitenkette verbunden sind. Unterschiedliche Strukturvarianten ergeben sich durch die Anordnung und Verknüpfung der C3-Seitenketten. Die biologischen Eigenschaften von Lignanen sind umfangreich. Es handelt sich um Phytoestrogene, also natürliche Pflanzenestrogene, die eine ausgleichende Wirkung auf den Hormonhaushalt haben. Darüber hinaus haben Lignane eine stark antioxidative Wirkung.

Erfindungsgemäß wird ein Extrakt aus Lignanen bevorzugt aus Leinsamen isoliert. Als Leinsamen werden die Samen des Flachs (Gemeiner Lein, *Linum usitatissimum*) bezeichnet. Lein liefert im Vergleich zu den meisten anderen Pflanzen das 75 bis 800fache an Lignanen. Sie weisen einen hohen Anteil von Secoisolariciresinol-Diglucoside (SDG) auf. Diese werden im Darm in zwei andere Lignane, nämlich Enterodiol und Enterolacton, umgewandelt, die gemäß mehrerer Studien einen gesundheitlichen Nutzen im Klimakterium bewirken. Man findet die beiden Abbauprodukte in verschiedenen Körperflüssigkeiten (Plasma, Speichel, Harn usw.), wo sie biologisch aktiv sind. Sie können die Estrogenrezeptoren erreichen. Der Gehalt an Enterolacton korreliert mit der Aufnahme pflanzlicher Isoflavonoide und Lignane. Lignane interferieren mit der Bindung von Testosteron, 5α-Dihydrotestosteron und Estradiol am Sexualhormonbindenden Globulin, wodurch eine Bindung jener Hormone möglich ist. Der gesundheitliche Nutzen von Lignanen zeigt sich laut Forschungsergebnissen auch in den Bereichen Prostatagesundheit, Knochengesundheit, Brustgesundheit, Linderung von Wechseljahrsbeschwerden, Herzgesundheit, Haarausfall, Akne, Entzündungen und potenzielle Krebsprävention. Ein erfindungsgemäßer Extrakt enthält vorzugsweise 5 bis 60%, insbesondere 10 bis 40 Gew.% Secoisolariciresinol-Diglucoside. Der Extrakt enthält bevorzugt mindestens 5, 10 oder 15 Gew.% Lignane.

### Granatapfel:

In einer bevorzugten Ausführungsform der Erfindung enthält die erfindungsgemäße Zusammensetzung einen pflanzlichen Extrakt aus Granatapfel. Bevorzugt werden dabei hohe Anteile an Polyphenolen und/oder Punicosiden extrahiert. Bevorzugt enthält ein erfindungsgemäßer Granatapfelextrakt mindestens 5, 10 oder 25 Gew.% Polyphenole und mindestens 5,10 oder 25 Gew.% Punicoside.

Der Granatapfel enthält eine Vielzahl von gesundheitsfördernden Wirkstoffen, so eine fast ausschließlich und in hoher Konzentration in dieser Frucht vorkommende Fettsäure, die Ellagsäure. Weitere gesundheitsfördernde Bestandteile sind Betacarotin, Vitamine, Mineralstoffe, Spurenelemente und große Mengen antioxidativer sekundärer Pflanzenstoffe, wie Polyphenole (Flavonoide, Tannine).

Die Wirkstoffe lindern Entzündungen und beschleunigen die Heilung von Wunden durch Regeneration des Gewebes. Granatapfelextrakt wirkt gegen Hautalterung, hat eine regenerative Wirkung auf die Epidermis, erhöht die Festigkeit und Elastizität der Haut, wirkt positiv ausgleichend auf die Hautfeuchtigkeit und ist faltenreduzierend. Es wurde insbesondere gezeigt, dass die Polyphenole und Punicoside des Granatapfels eine spezifische antiestrogene Wirkung besitzen.

Wegen seiner enormen Mengen an Phytoestrogenen kann der Granatapfel bei Frauen in der Menopause eine spürbare Linderung ihrer typischen Beschwerden bewirken. Neuere Studien zeigten, dass Granatapfelextrakt auch eine positive Wirkung in der Männermedizin hat und bei einer Prostatavergrößerung den typischen Beschwerden entgegenwirkt und das prostataspezifische Antigen (PSA) senken kann. Das Öl der Samen und der Blätter weist einen hohen Gehalt an Pflanzenhormonen auf. Die Fettsäuren des Granatapfels sind unter anderem für die Bildung von Prostaglandinen im menschlichen Körper verantwortlich. Prostaglandine sind für den Stoffwechsel und die Immunabwehr wichtig. Granatapfelschalen enthalten Bioflavonoide und Xanthone, die antiproliferativ, antientzündlich und antitumorös wirken. In Nanopartikel verkapselte Granatapfelextrakte sind sehr wirksam zur Behandlung von entzündlichen Hauterkrankungen wie Neurodermitis, Akne und Ekzeme.

Die Polyphenole des Granatapfels besitzen auch eine spezifische antiestrogene Wirkung (Van Elswijk et al., Phytochemistry, 2004; 65(2), 233-241) und dienen zur Linderung von Wechseljahresbeschwerden (Lansky et al., J. Ethnopharmacol 2007, Jan 19; 109: 177-206). Gesundheitsfördernde Effekte konnten auch gezeigt werden bei Vorbeugung gegen Brustkrebs und Osteoporose, bei Arteriosklerose, Bluthochdruck, Magenerkrankung, bakteriellen Infektionen, viralen Infektionen (HIV-1), Brustkrebs, Prostatakrebs (einschl. Beeinflussung des Anstiegs des prostataspezifischen Antigens PSA), Darmkrebs, Hautkrebs, Lungenkrebs und Diabetes mellitus.

### Traubensilberkerze

In einer bevorzugten Ausführungsform der Erfindung enthält die Zusammensetzung einen Extrakt der Traubensilberkerze (*Cimicifuga racemosa*). Dieser Stoff bewirkt eine Besserung der Wechseljahrbeschwerden und wird schon länger bei Beschwerden im Klimakterium eingesetzt. Ein erfindungsgemäß eingesetzter Extrakt aus Traubensilberkerze enthält bevorzugt mindestens 0,5, 1 oder 2 Gew.% Triterpene.

Die maßgebenden Stoffe gegen klimakterische Beschwerden aus der Traubensilberkerze sind die Triterpene (Triterpenglykoside, Cimicifugosid, Imiracemosid F, Actein, Cimicifugoside M2 und 27-Desoxyactein). Triterpene sind Phytoestrogene und Phytohormone, also pflanzliches Hormone, die in der Wirkung dem Estrogen ähnlich sind, obwohl sie chemisch unterschiedlich aufgebaut sind. Sie wirken teilweise etwas ähnlich wie weibliche Hormone und können dadurch verschiedene Frauenbeschwerden lindern. Triterpen ist ein selektiver Estrogen-Rezeptor-Modulator. Er wird mit Erfolg bei den typischen Beschwerden in den Wechseljahren eingesetzt. Im Klimakterium lässt die Leistung des Ovars zur Bildung von Estrogenen nach; Folgen sind Hitzewallungen, Kopfschmerzen und nächtliche Schweißausbrüche, weiterhin vaginale Änderungen und ein erhöhter Knochenabbau (Osteoporose). Als Standardtherapie hatte sich in den letzten Jahrzehnten die Hormonersatztherapie, also die Anwendung synthetischer Estrogene, insbesondere zur Vermeidung der erniedrigten Knochendichte und zur Reduktion der besonders beeinträchtigenden Hitzesymptome etabliert. In neueren Studien wurde jedoch herausgefunden, dass die Hormonersatztherapie mit einem Nebenwirkungsrisiko, wie der Tumorbegünstigung, einhergehen kann. Somit bietet sich die Traubensilberkerze als eine natürliche Alternative an. Man vermutet heute, dass an der Gesamtwirkung Effekte am Hyptothalamus als auch im Zusammenspiel mit Neurotransmittern beteiligt sind. In Studien wurde außerdem belegt, dass Traubensilberkerze die subjektiven Beeinträchtigungen verbessert, die Knochendichte (das Maß für die Osteoporose) erhöht, und auch das Scheidenmilieu verbessert.

### Mönchspfeffer:

In einer weiteren bevorzugten Ausführungsform der Erfindung enthält die Zusammensetzung einen Extrakt des Mönchspfeffers (*Agnus castus*)*.* Der Mönchspfeffer enthält Phytoestrogene und beseitigt eine Vielzahl von gynäkologischen Beschwerden und ist bekannt für seine ausgezeichnete Verträglichkeit. Mehrere Studien beschreiben die positive Wirkung für klimakterische Beschwerden. Ein vorteilhaftes Resultat zeigte die zweitgrößte placebokontrollierte Studie mit Mönchspfeffer, bei der Patientinnen, welche die diagnostischen Kriterien für ein prämenstruelles dysphorisches Syndrom erfüllten, während drei Zyklen Mönchspfeffer-Extrakt einnahmen. Die größten Verbessemngen wurden registriert bezüglich Reizbarkeit, Stimmungsveränderungen, Ärger und Kopfschmerzen. Diese Symptome sind auch im Klimakterium zu beobachten. In den USA wird *Agnus castus* bei klimakterischen Symptomen seit längerem eingesetzt.

Sowohl psychische, emotionale wie auch physische Symptome sprechen auf *Agnus castus* an. In weiteren Studien wurde ein Nutzen für Frauen bei mit zyklusabhängigen Schmerzen in den Brüsten und bei prämenstruellen Beschwerden, insbesondere prämenstruellem Spannungssyndrom und prämenstrueller dysphorischer Störung gezeigt. Ein bekanntes Mittel, das einen Mönchspfefferextrakt enthält, ist Agnolyt®.

Besonders bevorzugt ist eine erfindungsgemäße Zusammensetzung, die sowohl Extrakte von Traubensilberkerze als auch Mönchspfeffer enthält. Eine solche Kombination zeigt eine besondere Wirkung bei Wechseljahrbeschwerden, insbesondere psychovegetativer Symptomatik. Der Mönchspfeffer und die Traubensilberkerze ergänzen sich ideal mit den weiteren Wirkstoffen in der erfindungsgemäßen Formulierung.

### Acetyl-L-Carnitin:

In einer bevorzugten Ausführungsform der Erfindung enthält die erfindungsgemäße Zusammensetzung Acetyl-L-Carnitin (ALC). ALC erhöht cholinerge und serotonerge Aktivitäten, indem es die Nervenerregbarkeit auf diese beiden Nervenbotenstoffe (Acetylcholin und Serotonin) verbessert. Dabei werden sowohl die erregenden als auch die hemmenden Wirkungen von Serotonin erleichtert. Die Gabe von ALC ist ein sehr vorteilhafter Weg, den Serotonin-Stoffwechsel zu normalisieren, denn hier wird die Verstoffwechslung des Serotonin nicht manipuliert, sondern die Rezeptorsensibilität heraufreguliert. Somit ist insbesondere anzunehmen, dass ALC die Beschwerden von Frauen im Klimakterium mit chronischer Niedergeschlagenheit lindert oder beseitigt, die im Zusammenhang mit allgemeiner Rezeptor-Desensibilisierung stehen.

### Weitere Inhaltsstoffe:

In einer weiteren Ausführungsform der Erfindung können weitere Phytotherapeutika enthalten sein. Als "Phytotherapeutikum" werden allgemein aus Pflanzen isolierte gesundheitsfördernde Stoffe oder Stoffgemische bezeichnet, wie Antioxidantien oder Hormone.

Weitere geeignete weitere Inhaltsstoffe sind beispielsweise Mineralstoffe, wie Magnesium, Calcium, Vitamine und vitaminähnliche Stoffe (Vitaminoide, Pseudovitamine), Vitamin E oder C, Omega-3-Fettsäuren, insbesondere Coenzym Q10, Carnitin, Inositol und Cholin. Bevorzugte weitere Inhaltsstoffe sind insbesondere solche, die in Anhang 1 der Deutschen Nahrungsergänzungsmittelverordnung (NemV) aufgeführt.

### Verwendung:

Die Formulierung besteht zu einem hohen Anteil aus extrahierten pflanzlichen Inhaltstoffen. Diese zeigen eine neue, bislang nicht bekannte synergetische Wirkung, um Beschwerden, insbesondere im Klimakterium, entgegenzuwirken. Die Erfindung beruht auf dem überraschenden Befund, dass Equol die Wirkung weiterer Phytoestroge verstärken kann. Es ist bevorzugt, dass in der erfindungsgemäßen Zusammensetzung Phytoestrogene eingesetzt werden, die bereits für sich genommen wirksam gegen Beschwerden im Zusammenhang mit dem Klimakterium sind. Soll die erfindungsgemäße Zusammensetzung speziell gegen andere Indikationen wirken, so ist es entsprechend vorteilhaft, einzelne Phytoestrogene auszuwählen, für die bereits eine solche Wirkung bekannt ist.

Die erfindungsgemäße Zusammensetzung eignet sich allgemein zur Linderung und Beseitigung von Beschwerden und zur Prävention. Die Beschwerden sind insbesondere solche, die im Zusammenhang mit der Regulation des ERα- und ERβ-Rezeptors stehen. Die Verwendung kann dabei vorbeugend oder therapeutisch erfolgen. Die Verwendungen können therapeutisch in oder als Arzneimittel oder nicht als Arzneimittel, insbesondere als Nahrungsergänzungsmittel oder in einem Nahrungsmittel erfolgen. Erfindungsgemäß ist der Begriff "Behandlung" also nicht auf die Verwendung als Arzneimittel beschränkt.

Die erfindungsgemäße Zusammensetzung eignet sich insbesondere zur Behandlung oder Prävention von Beschwerden von Frauen im Zusammenhang mit dem Klimakterium. Grundsätzlich können in der erfindungsgemäßen Zusammensetzung Phytoestrogene eingesetzt werden, deren Wirkung bei der Behandlung von Beschwerden von Frauen in der Menopause bekannt ist (Dalais et al., Climacteric 1998, 1, 124-129). Die Verabreichung kann in den verschiedenen Stadien des Klimakteriums erfolgen, also in der Menopause, Pre-Menopause und Post-Menopause. Die Menopause ist bei der Frau das physiologische Aufhören der Menstruation, das die Fruchtbarkeit beendet. Sie ist verursacht durch eine nach und nach abnehmende Funktion der Eierstöcke. Die Menopause kann natürlich oder vorzeitig eintreten oder künstlich herbeigeführt werden. Festgestellt wird sie gewöhnlich, wenn ein Jahr lang keine Menstruationsblutungen mehr aufgetreten sind. Die Übergangsphase der hormonellen Umstellung, die in den Jahren davor und danach stattfindet, wird als Klimakterium (Wechseljahre) bezeichnet, manchmal wird dafür aber auch der Ausdruck Menopause verwendet. Die natürliche Menopause tritt gewöhnlich zwischen dem 45. und 55. Altersjahr ein, durchschnittlich im Alter von 50 bis 51 Jahren. Als vorzeitige Menopause (*Climacterium praecox*) wird eine Menopause durch mangelndes Funktionieren der Eierstöcke vor dem 40. Lebensjahr bezeichnet. Die Menopause als solche ist keine Krankheit. Eine Behandlung kann jedoch bei Beschwerden nötig sein, die durch die hormonelle Umstellung im Klimakterium entstehen.

Beschwerden im Zusammenhang mit dem Klimakterium, bei denen die erfindungsgemäße Zusammensetzung einsetzt werden kann, sind insbesondere Migräne, Osteoporose, Stimmungsveränderungen, Ärger, Reizbarkeit, Kopfschmerzen, Schlafstörungen, Hitzewallungen, Schwitzen, nächtliche Schweißausbrüche, Herzklopfen, chronische Niedergeschlagenheit (Depressionen), Veränderungen der Vagina und Libidoverlust. Die Zusammensetzung zeigt bei Verabreichung im Klimakterium eine günstige Beeinflussung der kognitiven Fähigkeiten, Verbesserung der mentalen Leistungsbereitschaft (Kurz-, Langzeitgedächtnis, Reaktivität) sowie eine antioxidative Wirkung.

Beschwerden im Zusammenhang mit dem Klimakterium und der Menopause können klinisch unterschieden werden von ähnlichen Beschwerden, die andere Ursachen haben, beispielsweise durch Krankheiten verursacht sind. Beschwerden im Zusammenhang mit dem Klimakterium sind insbesondere solche, die mit Veränderungen des Stoffwechsels einhergehen und klinisch manifestierbar sind. Im Allgemeinen zeigt sich dabei eine veränderte Aktivität der Estrogenrezeptoren.

Die erfindungsgemäße Zusammensetzung kann darüber hinaus bei Beschwerden und Krankheiten eingesetzt werden, die nicht im Zusammenhang mit dem Klimakterium stehen. Sie kann eingesetzt werden gegen Entzündungen, Wunden durch Regeneration des Gewebes, Neurodermitis, Akne und Ekzemen, entzündliche Hauterkrankungen, Hautalterung, Haarausfall, Akne, Arteriosklerose, Bluthochdruck, Magenerkrankungen, bakterielle Infektionen, virale Infektionen wie HIV-1, Brustkrebs, Prostatakrebs, Darmkrebs, Hautkrebs, Lungenkrebs, Diabetes mellitus, zyklusabhängigen Schmerzen in den Brüsten, prämenstruellen Beschwerden, zur Verbesserung von Prostatagesundheit, Knochengesundheit, Brustgesundheit, Herzgesundheit, Stoffwechsel, Immunabwehr, Krebsprävention, Regeneration der Epidermis, Erhöhung der Festigkeit und Elastizität der Haut, Ausgleichung der Hautfeuchtigkeit, zur Reduzierung von Falten oder zur antiproliferativ, antientzündlich und antitumorös Behandlung.

Gegenstand der Erfindung ist auch ein Verfahren zur Herstellung einer erfindungsgemäßen, wobei gereinigtes Equol (a) mit dem pflanzlicher Extrakt von Isoflavonen (b) und gegebenenfalls weiteren Inhaltsstoffen vermischt wird. Das Equol kann einen vorhergehenden Schritt umfassen, bei dem dass Equol hergestellt und/oder gereinigt wird. Nach dem Vermischen wird die Zusammensetzung gegebenenfalls konfektioniert, portioniert und/oder verpackt.

### Ausführungsbeispiele:

Es wurde die Wirkung einer erfindungsgemäßen Zusammensetzung auf Probanden untersucht. Das erfindungsgemäße Produkt hatte folgende Zusammensetzung:
80 mg Equol
120 mg Isoflavone aus Soja (97 Gew.% Isoflavone)
350 mg Lignan-Extrakt aus Leinsamen (15 Gew.% Secoisolariciresinol-Diglucoside)
400 mg Granatapfel (50 Gew.% Polyphenole und 40 Gew.% Punicoside)
400 mg Traubensilberkerze (*Cimifuga racemosa*, 4 Gew.% Triterpene)
300 mg Mönchspfeffer (*Agnus castus*)
150 mg Acetyl-L-Carnitin.

Es wurden die Expression von ERβ und die Unterdrückung des ERα gemessen. Es wurde zeitgleich die Ausschüttung der Exosomen, von Interkleukin-6 (IL-6), Tumomekrosefaktor (TNF) und C-reaktives Protein (CRP) gemessen. Die Werte wurden bei 5 Probanden bestimmt. Davon waren 3 weiblich und 2 männlich. Es wurden jeweils vier Werte gemessen, nämlich der Normalwert (1.), der Ausgangswert vor Verabreichung (2.), der Wert zwei Monate nach Verabreichung (3.) und der Wert vier Monate nach Verabreichung (4.).

In einem weiteren Versuch wurde geprüft, ob die beobachteten Effekte auf das gereinigte Equol zurückgeführt werden können. Dafür wurde eine Testreihe A mit der obigen Zusammensetzung ohne Equol durchgeführt, die als Kontrolle dient. In der Testreihe Gruppe B ist Equol gemäß der obigen Zusammensetzung enthalten.

### Ergebnisse:

Die gemessenen Werte sind in den Tabellen 1 und 2 aufgeführt. Die Zusammensetzung beinhaltet mehrere Wirkstoffe, die in großen Mengen und in hoher Reinheit verfügbar sind. Die Wirkstoffkombination beinhaltet auch gereinigtes Equol. Die Ergebnisse zeigen, dass durch die Zugabe von Equol die Verfügbarkeit der weiteren aktiven Wirkstoffe signifikant verbessert wird. Die Zusammensetzung verändert die Estrogenrezeptoren ERα und ERβ positiv. Der Estrogenrezeptor ERβ konnte in allen Probanden signifikant hochreguliert werden und auch der antagonistische Estrogenrezeptor ERα konnte entsprechend gehemmt werden. Die Estrogenwerte verbesserten sich in der Equol Gruppe deutlich. Die Zusammensetzung ist daher bei der Regulation dieser Rezeptoren bei klimakterischen Beschwerden einsetzbar. Insgesamt wird die Wirksamkeit der aktiven Substanzen die Zugabe von Equol signifikant verbessert. Der Estrogenrezeptoren Beta (ERβ) konnte stark hochreguliert werden. Der Estrogenrezeptoren Alpha (ERα) konnte signifikant herunterreguliert werden. Die Estrogenwerte konnte signifikant verbessert werden.

Verbesserungen wurden auch im Bereich der Exosomen festgestellt. Auch die Inflammationswerte von IL-6 zeigten positive Veränderungen. Die Werte TNF (Tumornekrosefaktor) und CRP (Capsel-reactive Protein) wurden ebenfalls positiv moduliert.

**Tabelle 1:**

| | **Messung** | **1.** | **2.** | **3.** | **4.** |
|---|---|---|---|---|---|
| | | | | | |
| **W 30 J** | Exosome Elisspot | 0,7 | 1,3 | 1,5 | 0,9 |
| | ER-β | 300 | 217 | 230 | 265 |
| | ER-α | 0,9 | 0,8 | 0,8 | 0,6 |
| | IL-6 Immulite DPC pg/ml | 9,7 | 10,3 | 10,1 | 9,7 |
| | TNF Immulite DPC pg/ml | 8,1 | 10,1 | 8,9 | 7,4 |
| | CRP Kryptor Brahms µg/ml | 5 | 4 | 3,8 | 3,7 |
| | | | | | |
| **W 55 J** | Exosome Elisspot | 0,7 | 2,3 | 1,8 | 1,3 |
| | ER-β | 300 | 241 | 269 | 460 |
| | ER-α | 0,9 | 1,2 | 1,4 | 0,6 |
| | IL-6 Immulite DPC pg/ml | 9,7 | 24 | 19 | 15 |
| | TNF Immulite DPC pg/ml | 8,1 | 24 | 23 | 17 |
| | CRP Kryptor Brahms µg/ml | 5 | 28 | 17 | 9 |
| | | | | | |
| **M 48 J** | Exosome Elisspot | 0,7 | 3,3 | 2,1 | 1,3 |
| | ER-β | 300 | 177 | 197 | 278 |
| | ER-α | 0,9 | 1,6 | 1,5 | 1,4 |
| | IL-6 Immulite DPC pg/ml | 9,7 | 6 | 9 | 8 |
| | TNF Immulite DPC pg/ml | 8,1 | 35 | 22 | 18 |
| | CRP Kryptor Brahms µg/ml | 5 | 200 | 89 | 91 |
| | | | | | |
| **M 56 J mit Morbus crohn** | Exosome Elisspot | 0,7 | 0,6 | 0,7 | 0,5 |
| | ER-β | 300 | 180 | 379 | 398 |
| | ER-α | 0,9 | 1,7 | 0,6 | 0,4 |
| | IL-6 Immulite DPC pg/ml | 9,7 | 345 | 278 | 134 |
| | TNF Immulite DPC pg/ml | 8,1 | 24 | 23 | 21 |
| | CRP Kryptor Brahms µg/ml | 5 | 5 | 7 | 4 |
| | | | | | |
| **W 66 J** | Exosome Elisspot | 0,7 | 2,5 | 2,3 | 1,8 |
| | ER-β | 300 | 99 | 476 | 420 |
| | ER-α | 0,9 | 2,1 | 0,4 | 0,3 |
| | IL-6 Immulite DPC pg/ml | 9,7 | 38 | 37 | 25 |
| | TNF Immulite DPC pg/ml | 8,1 | 86 | 67 | 45 |
| | CRP Kryptor Brahms µg/ml | 5 | 359 | 276 | 198 |

**Tabelle 2:**

| Gruppe B | | 1. | A | B |
|---|---|---|---|---|
| 10 W Alter>35-40 | Oestradio pg/ml | 97 | 110 | 144 |
| 10 W Alter>45-50 | Oestradio pg/ml | 34 | 67 | 89 |

| | | | | |
|---|---|---|---|---|
| Erläuterungen: W = weiblich, M = männlich, J = Jahre 1. Normalwerte, Gesunde Ausgangsgruppe (17-20 J) 2 M 2 F 2. Grundwerte vor Therapie 3. nach 2 Monaten 4. nach 4 Monaten A nach 3 Monaten ohne Equol B: nach 3 Monaten mit Equol | | | | |

## Patentansprüche

1. Zusammensetzung, enthaltend
(a) gereinigtes Equol und
(b) mindestens ein weiteres Phytoestrogen.

2. Zusammensetzung nach Anspruch 1, die keine Enzyme oder Mikroorganismen enthält, die Equol herstellen.

3. Zusammensetzung nach Anspruch 1 oder 2 wobei das mindestens eine Phytoestrogen (b) ein pflanzlicher Extrakt ist.

4. Zusammensetzung nach einem der vorhergehenden Ansprüche, wobei das mindestens eine Phytoestrogen (b) ausgewählt ist aus Isoflavonen, insbesondere Extrakten aus Soja, Lignanen, insbesondere Extrakten aus Leinsamen, sowie Extrakten aus Traubensilberkerze, Granatapfel oder Mönchspfeffer, oder einem Gemisch davon.

5. Zusammensetzung nach einem der vorhergehenden Ansprüche, wobei als Phytoestrogen (b1) ein Extrakt von Isoflavonen, insbesondere aus Soja, sowie mindestens ein weiteres Phytoestrogen (b2), ausgewählt aus Extrakten aus Traubensilberkerze, Granatapfel oder Mönchspfeffer und Extrakten von Lignanen, insbesondere aus Leinsamen, enthalten sind.

6. Zusammensetzung nach einem der vorhergehenden Ansprüche, enthaltend 0,5 bis 30 Gew.-% gereinigtes Equol.

7. Zusammensetzung nach einem der Ansprüche 4 bis 6, wobei das Gewichtsverhältnis des gereinigten Equols (a) zu dem Extrakt von Isoflavonen (b1) zwischen 1:10 und 5:1 ist.

8. Zusammensetzung nach einem der vorhergehenden Ansprüche, in der ausschließlich R-Equol oder S-Equol enthalten ist.

9. Zusammensetzung nach einem der vorhergehenden Ansprüche, wobei als Phytoestrogene (b) enthalten sind:
(b1) ein pflanzlicher Extrakt von Isoflavonen,
(b2) ein pflanzlicher Extrakt von Lignanen und
(b3) mindestens ein weiterer Pflanzenextrakt, ausgewählt aus Traubensilberkerze, Granatapfel und Mönchspfeffer.

10. Zusammensetzung nach einem der vorhergehenden Ansprüche, wobei zusätzlich (c) mindestens ein Aminosäurederivat oder eine Aminosäure enthalten ist.

11. Zusammensetzung nach einem der vorhergehenden Ansprüche, enthaltend
(a) 0,5 bis 30 Gew.-% gereinigtes Equol,
(b1) 1 bis 60 Gew.-% pflanzlicher Extrakt von Isoflavonen,
(b2) 1 bis 30 Gew.-% pflanzlicher Extrakt von Lignanen,
(b3) 1 bis 90 Gew.-% weitere Pflanzenextrakte und
(c) 0 bis 20 Gew.-% Aminosäurederivate.

12. Zusammensetzung nach einem der Ansprüche 1 bis 11 in Form eines Nahrungsergänzungsmittels oder eines Arzneimittels.

13. Nahrungsmittel, Nahrungsergänzungsmittel oder Arzneimittel, enthaltend eine Zusammensetzung nach einem der Ansprüche 1 bis 12.

14. Verwendung einer Zusammensetzung nach einem der Ansprüche 1 bis 12 oder eines Nahrungsmittels, Nahrungsergänzungsmittels oder eines Arzneimittels nach Anspruch 13 zur Behandlung von klimakterischen Beschwerden von Frauen, gegen Entzündungen, Wunden durch Regeneration des Gewebes, Neurodermitis, Akne und Ekzemen, entzündliche Hauterkrankungen, Hautalterung, Haarausfall, Akne, Arteriosklerose, Bluthochdruck, Magenerkrankungen, bakterielle Infektionen, virale Infektionen wie HIV-1, Brustkrebs, Prostatakrebs, Darmkrebs, Hautkrebs, Lungenkrebs, Diabetes mellitus, zyklusabhängigen Schmerzen in den Brüsten, prämenstruellen Beschwerden, zur Verbesserung von Prostatagesundheit, Knochengesundheit, Brustgesundheit, Herzgesundheit, Stoffwechsel, Immunabwehr, Krebsprävention, Regeneration der Epidermis, Erhöhung der Festigkeit und Elastizität der Haut, Ausgleichung der Hautfeuchtigkeit, zur Reduzierung von Falten oder zur antiproliferativ, antientzündlich und antitumorös Behandlung.

15. Verfahren zur Herstellung einer Zusammensetzung nach einem der Ansprüche 1 bis 12, wobei gereinigtes Equol (a) mit dem pflanzlicher Extrakt von Isoflavonen (b) und gegebenenfalls weiteren Inhaltsstoffen vermischt wird.
